# EUROPÄISCHE PATENTSCHRIFT

(11) **EP 1 078 290 B1**
(45) Veröffentlichungstag und Bekanntmachung des Hinweises auf die Patenterteilung: **21.05.2003**
(21) Anmeldenummer: 99917907.0
(22) Anmeldetag: 25.03.1999
(51) Int. Cl.: G02B 21/08

(54) **BELEUCHTUNGSEINRICHTUNG FÜR EIN OPERATIONSMIKROSKOP**
LIGHTING DEVICE FOR A SURGICAL MICROSCOPE
DISPOSITIF D'ECLAIRAGE POUR MICROSCOPE OPERATOIRE

(30) Priorität: 13.05.1998 CH 107898
(43) Veröffentlichungstag der Anmeldung: 28.02.2001
(73) Patentinhaber: Leica Microsystems (Schweiz) AG, 9435 Heerbrugg (CH)
(72) Erfinder: DEVERIN, Jacques, Alain, CH-9443 Widnau (CH)
(74) Vertreter: Reichert, Werner F., Dr.
(86) Internationale Anmeldenummer: EP9902151
(87) Internationale Veröffentlichungsnummer: WO99059016

(56) Entgegenhaltungen:
- DE-A- 19 650 773
- DE-U- 9 314 578
- FR-A- 2 666 662
- PATENT ABSTRACTS OF JAPAN vol. 097, no. 008, 29. August 1997 & JP 09 105866 A (OLYMPUS OPTICAL CO LTD), 22. April 1997 in der Anmeldung erwähnt

## Beschreibung

Die Erfindung betrifft eine Beleuchtungseinrichtung für ein Operationsmikroskop. Im Bereich der Mikroskopie sind verschiedenste Beleuchtungseinrichtungen bekannt geworden. Besonders bei der Operationsmikroskopie - und da insbesondere bei Operationsmikroskopen für die Ophtalmologie - ist die Beleuchtung ein besonders heikles Thema, da einerseits die optimale Beleuchtung für den Chirurgen erfolgentscheidend sein kann und andererseits hinsichtlich der Gefährdung des Gewebes unter dem Mikroskop nicht mit beliebigen Lichtstärken beleuchtet werden kann.

Die DE-C2- 40 28 605 und ihr Familienmitglied US-A- 5,126,877 stellen eine bekannte Beleuchtungseinrichtung vor. Eine noch ältere, an sich gut funktionierende Beleuchtungseinrichtung wurde durch die Vorgängerin der Anmelderin unter der Bezeichnung "Beleuchtungsoptik 0°" bereits um 1985 auf den Markt gebracht (vgl. Prospekt M1 668d-X.85 aus Oktober 1985 der Wild Heerbrugg AG). Weitere relevante Beleuchtungs einrichtungen sind aus der DE 93 14 578.0 U und DE 196 50773 bekannt.

Bei diesem Aufbau wird der gesamte Lichtstrahlengang aus einer, einer Lampe zugeordneten Beleuchtungsoptik durch ein erstes Prisma aus einer Senkrechten auf die Hauptachse des Hauptobjektivs des Mikroskops in eine Schräge mit kleinem Winkel (z.B. 6°) zur Hauptachse umgelenkt. Dieses stellt die Hauptbeleuchtung des Operationsfeldes dar. Um aber zu der - insbesondere bei Augenoperationen hinsichtlich des sich daraus ergebenden "Redreflex" gewünschten - "0°-Beleuchtung" zu kommen, ist zwischen dem ersten Prisma und dem Hauptobjektiv eine zur Hauptebene des Hauptobjektivs parallele transparente Scheibe vorgesehen. Diese ist um die Hauptachse drehbar. Die Scheibe trägt ein zweites - im Vergleich zum ersten relativ schmales - Prisma, das einen Teil des durch das erste Prisma umgelenkten Lichtstrahlenganges über zwei annähernd parallele Spiegelflächen in die Hauptachse legt.

Durch das Drehen der Scheibe kann somit wahlweise zu einer Standardbeleuchtung in einem kleinen Winkel zur Hauptachse eine "0°-Beleuchtung" hinzugefügt werden, die im gleichen Ausmass ihrer Lichtstromzunahme den Lichtstrom der Standardbeleuchtung reduziert. Theoretisch ist dabei eine Steuerung der Lichtstromverteilung über die Winkelstellung um die Hauptachse möglich.

So bewährt dieser bekannte Aufbau ist, so sehr wurde nach neueren Lösungen gesucht, die ohne rotierende Scheiben auskommen. Als eine solche Lösung wurde die Eingangs erwähnte - gemäss der zitierten DE-C und der US-A angegeben.

Ein noch wesentlich jüngerer bekannter Aufbau ist in der JP-A-9-105866 angegeben. Etwa vergleichbar mit dem alten Aufbau "0°-Beleuchtung" der Vorgängerin der Anmelderin findet sich dort unter einem ersten Prisma ein zweites Prisma mit zwei Spiegelflächen, das allerdings nicht um die Hauptachse drehbar ist. Dieser Aufbau führt zu einer grundsätzlich konstanten Lichtstromaufteilung zwischen dem 0°-Lichtstromteil und jenem grösseren, der permanent unter kleinem Winkel einfällt, wobei anscheinend eine gemeinsame Reduktion des Lichtstromes durch eine einschiebbare Blende im Lichtstrahlengang der Beleuchtungsoptik möglich ist.

Dieser japanische Aufbau hat mit der "0°-Beleuchtung" gemeinsam, dass zwei Prismen hinter einander angeordnet sind und beide vor dem Hauptobjektiv somit eine bestimmte Bauhöhe erzwingen. Auch führt zwingend die Regulierung des Lichtstromes im Bereich der Hauptachse zu Änderung des Lichtstromes, der unter dem kleinen Winkel auf das Objekt fällt.

Sowohl der eine, als bevorzugt auch der andere Effekt sollte erfindungsgemäss vermieden werden.

Demzufolge liegt der Erfindung die Aufgabe zugrunde, eine kompakte Beleuchtungseinrichtung mit je wenigstens einem - in bezug auf die Hauptachse - schrägen und wenigstens einem parallelen Lichtstrom zu schaffen, bei der möglichst beide Lichtströme voneinander unabhängig reguliert werden können.

Gelöst wird diese Aufgabe erstmals durch den Aufbau gemäss Anspruch 1.

Die Kombination der Merkmale des Anspruches 1 führt zu einer geringen Bauhöhe, da die Prismen nunmehr nebeneinander sind und stellt zudem sicher, dass die Lichteintrittsflächen beider Prismen voneinander unabhängig mit einem Teil-Lichtstrom versorgt werden, so dass jeder dieser Teillichtströme vom anderen unabhängig reguliert werden kann.

Ein Beispiel einer solchen Regulationsmöglichkeit ist in Anspruch 2 angegeben. Einfach verstellbare Blenden sind in Anspruch 3 angegeben. Eine besonders anwenderfreundliche Blende ist durch die Merkmale des Anspruches 4 beschrieben. LCD's können einerseits durch selektive Abdunkelung als Graufilter eingesetzt werden, andererseits können elektronisch abgespeicherte Fensterformen freigebbar sein, die die verschiedensten gewünschten Beleuchtungseinstellungen abdecken.

Die Erfindung ist jedoch auf diese angegebenen Blenden nicht eingeschränkt, sondern kann vielmehr mit jeder bekannten oder neuen Lichtreguliervorrichtung ausgerüstet sein, ohne ihr Wesen, nämlich die flache, kompakte Bauart und die unabhängige Regulierbarkeit zu verlieren.

Eine besondere Ausgestaltung und gegebenenfalls eine Alternative zu dem bisher Beschriebenen, die alternativ oder auch in Kombination sinnvoll angewendet werden kann und bei Bedarf zudem zu einer verbesserten noch symmetrischeren Beleuchtung führt, ist in Anspruch 5 angegeben.

Unter einem Lichtbrechteil sind alle jene Bauteile zu verstehen, die im Kontakt mit einer Totalreflexionsfläche und an der Kontaktstelle deren Totalreflexion aufheben. Beispielsweise können dies Prismen, Glasplatten oder auch bestimmte - gegebenenfalls nichttransparente - Kunststoff oder Metallplatten (siehe Anspruch 6) oder Flüssigkeiten sein. Durch diese besondere Ausgestaltung der Erfindung sind nämlich einerseits die Möglichkeit zum Lichtregulieren an sich umfasst, andererseits aber auch die Möglichkeit zum Aufteilen eines Lichtstromes für die Anwendung eines weiteren optionalen Spiegelelementes zur Erzeugung eines weiteren Beleuchtungsstrahlenganges in einem Winkel zur Hauptachse, wie in Anspruch 7 angegeben.

Anspruch 8 definiert eine bevorzugte Ausführungsform, bei der mit Hilfe der Brechung der Totalreflexion selektiv nur die "0°-Beleuchtung" geregelt wird bzw. im Falle des Abzweigens von Lichtstrom aus dem zweiten Prisma, dieser einer von der ersten schrägen Beleuchtung unabhängigen weiteren schrägen Beleuchtung zugeführt wird.

Unabhängig oder zusätzlich zur Lichtstromregelung kann erfindungsgemäss auch eine Lichtfarbenregelung vorgesehen sein, wie in Anspruch 9 angegeben.

Anhand der Zeichnung wird die Erfindung beispielhaft näher erläutert.Es zeigen dabei die
Fig.1 die Draufsicht auf die wesentlichen Elemente der Erfindung;
Fig.2 die erweiterte Seitansicht mit schematischer Beleuchtungsoptik und Hauptobjektiv und
Fig.3 ein Beispiel einer erfindungsgemässen Blendenscheibe.

Die Figuren werden zusammenhängend beschrieben. Gleiche Bezugszeichen bedeuten gleiche Bauteile. Gleiche Bezugszeichen mit unterschiedlichen Indizes bedeuten Teile mit gleichen Aufgaben bzw. ähnlichen Funktionen.

Ein zweigeteiltes erstes Prisma 4a und 4b flankiert ein zweites Prisma 5. Beispielsweise sind diese miteinander verklebt. Alle Prismen haben je eine Lichteintrittsfläche 7, die einer Beleuchtungsoptik 2 zugewandt ist. Beispielshaft ist eine Lichtquelle 3 dargestellt, die auch gut einen Lichtwellenleiter umfassen könnte. Die Zweiteilung des Prismas 4 ist bevorzugt, da sich dadurch eine gute Symmetrie bei der Ausleuchtung am Objekt ergibt.

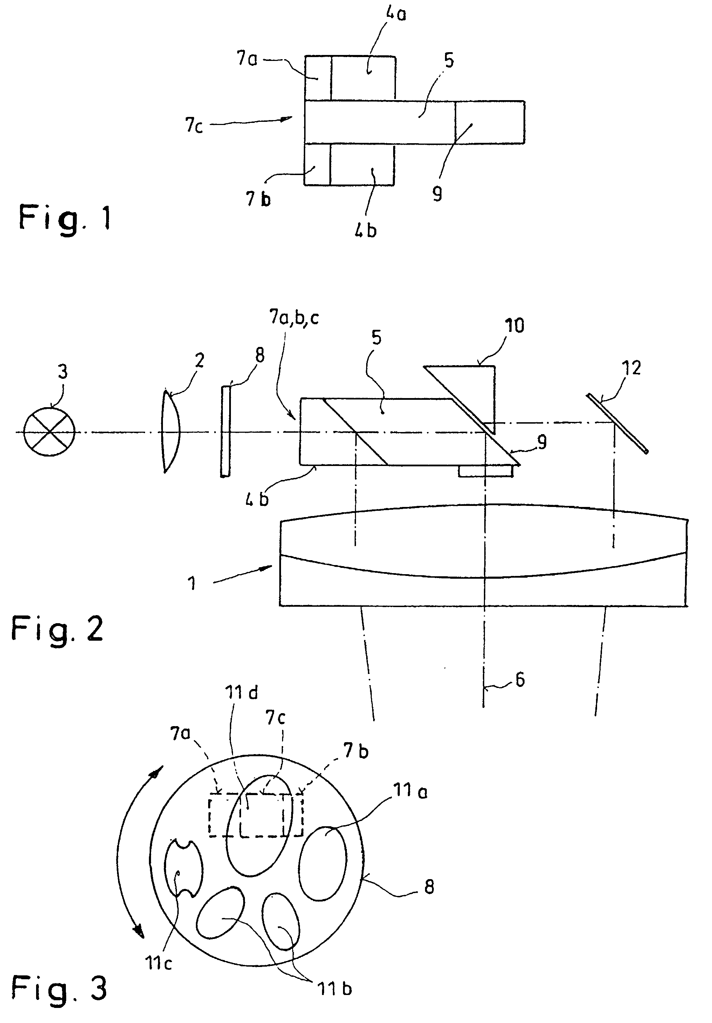

Zwischen der Beleuchtungsoptik 2 und den Lichteintrittsflächen 7 ist optional eine verstellbare Blende 8 angeordnet. Diese kann beispielsweise so aussehen, wie in Fig.3 dargestellt. Dort erkennt man Fenster 11a-11d, die wahlweise vor die Lichteintrittsflächen 7 gestellt werden können. In Fig.3 ist das Fenster 11d vor die Lichteintrittsfläche 7c des zweiten Prismas gesetzt, so dass in diesem Fall nur die 0°-Beleuchtung mit voller Lichtstärke aktiviert ist. Die beiden ersten Prismenteile 4a und 4b erhalten praktisch keinen Lichtstrom. Würde man das Fenster 11d etwas weiterdrehen, so könnte dadurch eine Aufteilung des Lichtstromes zwischen der Lichteintrittsfläche 7c und einer von beiden Lichteintrittsflächen 7a oder 7b erfolgen.

Als krasse Alternative sind die Fenster 11b vorgesehen, die eine vollständige Abdunkelung der 0°-Beleuchtung bei gleichzeitiger Vollbeleuchtung des ersten Prismas 4 ermöglichen. Fenster 11a erlauben eine Vollbeleuchtung aller Prismen 4,5 und Fenster 11c erlaubt eine Vollbeleuchtung des Prismas 5 bei gleichzeitiger Reduktion der Beleuchtung des ersten Prismas 4.

Die Prismen 4a,b könnten auch durchgängig einstückig ausgebildet sein, so dass die gesamte Eintrittsfläche 7a,b,c durch das Prisma 4 abgedeckt ist. Das Prisma 5 wäre dann als schmäleres Prisma an die Hypothenuse des Prismas 4 geklebt. Selbstverständlich können die Prismen 4 und 5 auch zusammen als einstückiges Prisma ausgebildet sein.

Unabhängig von der Blende 8 ist ein verschiebbares Prisma als Lichtbrechteil 10 vorgesehen, das mit einer planen Fläche an der Totalreflexionsfläche 9 des zweiten Prismas 5 verschiebbar ist. Wie nicht näher dargestellt, liegt es beispielsweise federbelastet an der Fläche 9 an. Durch die Unterbrechung der Totalreflexion wird ein Teil des Lichtstromes aus dem zweiten Prisma 5 nicht durch das Hauptobjektiv 1 gespiegelt sondern durch den Lichtbrechteil 10 aus dem zweiten Prisma 5 abgeleitet.

Unterstützend könnte hier ein Flüssigkeitsfilm, insbesondere ein Ölfilm auf der Kontaktfläche sein. Das könnte bei einer nichtgezeigten Variante eine Lichtvemichtung zur Folge haben, z.B. wenn die im Bild rechte Fläche des Lichtbrechteiles 10 schwarz gefärbt ist, oder wenn diese Fläche zwar durchsichtig ist, jedoch gegen eine schwarze Innentubusbeschichtung gerichtet ist.

Die Totalreflexion kann im Rahmen der Erfindung auch durch elektrooptische Schichten (z.B. LCD, Kristall oder aufgedampfte Schichten) zwischen den beiden Prismen gebrochen werden. Diese Schichten können - sofern sie elektronisch erregt werden selektiv erregt werden, um derart die gewünschten Bereiche totalreflektierend bzw. reflexionsbrechend zu gestalten.

Beim vorliegenden Ausführungsbeispiel ist diese Fläche jedoch gegen eine weitere Spiegelfläche 12 gerichtet, die den abgezweigten Lichtstrom wiederum in einem kleinen Winkel durch das Hauptobjektiv 1 gegen das Objekt lenkt. Bei Bedarf könnte die weitere Spiegelfläche noch verstellbar sein, um den Reflexionswinkel zu beeinflussen, wie jedoch nicht näher dargestellt ist.

## Patentansprüche

1. Beleuchtungseinrichtung für ein Operationsmikroskop mit einem Hauptobjektiv (1) um eine Hauptachse (6), bestehend aus einer Beleuchtungsoptik (2) mit einer Lichtquelle (3), und zwei Umlenkelementen, die zwischen dem Hauptobjektiv (1) und der Beleuchtungsoptik (2) vorgesehen sind, von denen im Betriebszustand das erste Umlenkelement die Umlenkung eines Teils des Lichtstromes aus der Beleuchtungsoptik (2) in einem kleinen Winkel zur Hauptachse (6) und das zweite Umlenkelement die Umlenkung eines anderen Teils des Lichtstromes aus der Beleuchtungsoptik (2) in die Hauptachse (6) bewirkt, wobei
die Umlenkelemente als Prismen (4,5) vorgesehen sind, das erste Prisma (4a,4b) des ersten Umlenkelements zweigeteilt ist und somit drei Prismen (4a,4b,5) vorgesehen sind,
die beiden Teile des ersten Prismas (4a,4b) des ersten Umlenkelements symmetrisch und seitlich zum zweiten Prisma (5) des zweiten Umlenkelements anliegen, um die symmetrische Ausleuchtung des Objektes zu bewirken die drei Prismen (4a,4b,5) neben einander in einer Ebene liegen, um eine niedrige Bauhöhe zu bewirken
und die Lichteintrittsöffnungen (7) der drei Prismen (4a,4b,5) zusammen den Ausgang der Beleuchtungsoptik (2) abschließen.

2. Beleuchtungseinrichtung nach Anspruch 1, **dadurch gekennzeichnet, dass** zwischen der Beleuchtungsoptik (2) und wenigstens einer Eintrittsfläche (7) wenigstens eines der Prismen (4, 5) wenigstens eine Iris-Blende (8) angeordnet ist.

3. Beleuchtungseinrichtung nach Anspruch 2, **dadurch gekennzeichnet,dass** die Blende (8) eine an sich als lichtundurchlässige Scheibe mit selektiv vorschaltbaren Fenstern (11) aufgebaut ist, die - dem Bedarf entsprechend - den Beleuchtungsoptikstrahlengang auf unterschiedliche Eintrittsflächen (7) der Prismen (4a, 4b, 5) in unterschiedlichem Umfang freigeben.

4. Beleuchtungseinrichtung nach Anspruch 2, **dadurch gekennzeichnet, dass** die Blende (8) als LCD aufgebaut ist, das - elektronisch angesteuert - frei wählbare Anteile der Eintrittsflächen (7) der Prismen (4a, 4b, 5) freistellt.

5. Beleuchtungseinrichtung nach einem der vorhergehenden Ansprüche, **dadurch gekennzeichnet, dass** wenigstens eine der Spiegelflächen (9) wenigstens eines Prismas (4,5) als Totalreflexionsfläche ausgebildet ist, und dass an wenigstens dieser Spiegelfläche (9) wenigstens ein Lichtbrechteil (10) totalreflexionsbrechend verschiebbar oder verschwenkbar oder elektrooptisch, elektronisch steuerbar angeordnet ist.

6. Beleuchtungseinrichtung nach Anspruch 5, **dadurch gekennzeichnet, dass** der Lichtbrechteil (10) als transparentes Prisma, als Glasplatte oder als lichtundurchlässiger Bauteil aufgebaut ist.

7. Beleuchtungseinrichtung nach Anspruch 5 oder 6, **dadurch gekennzeichnet, dass** der Spiegelfläche (9) gegenüber eine weitere Spiegelfläche (12) angeordnet ist, die aus dem Lichtbrechteil (10) austretende Strahlen in einem Winkel zur Hauptachse (6) umlenkt.

8. Beleuchtungseinrichtung nach einem der Ansprüche 5 bis 7, **dadurch gekennzeichnet, dass** die totalreflektierende Spiegelfläche (9) am zweiten Prisma (5) ausgebildet ist.

9. Beleuchtungseinrichtung nach einem der Ansprüche 2-8, **dadurch gekennzeichnet, dass** vergleichbar de Blende (8) zusätzlich oder alternativ ein selektives Farbwahlfilter vorgesehen ist.

## Claims

1. Illuminating device for a surgical microscope, having a main objective (1) about a main axis (6), comprising an illuminating optics (2) with a light source (3), and two deflecting elements which are provided between the main objective (1) and the illuminating optics (2), of which in the operating state the first deflecting element effects the deflection of a portion of the light flux from the illuminating optics (2) at a small angle to the main axis (6), and the second deflecting element effects the deflection of another portion of the light flux from the illuminating optics (2) into the main axis (6), in which the deflecting elements are provided as prisms (4, 5), the first prism (4a, 4b) of the first deflecting element is bipartite and three prisms (4a, 4b, 5) are thereby provided, the two parts of the first prism (4a, 4b) of the first deflecting element symmetrically and laterally adjoin the second prism (5) of the second deflecting element in order to effect the symmetrical illumination of the object, the three prisms (4a, 4b, 5) lie next to one another in a plane, so as to effect a low overall height, and the light entry openings (7) of the three prisms (4a, 4b, 5) jointly terminate the exit of the illuminating optics (2).

2. Illuminating device according to Claim 1, **characterized in that** at least one iris diaphragm (8) is arranged between the illuminating optics (2) and at least one entrance surface (7) of at least one of the prisms (4, 5).

3. Illuminating device according to Claim 2, **characterized in that** the diaphragm (8) per se is constructed as an opaque disk with windows (11) which can be selectively inserted upstream and which - in accordance with the requirement - uncover to a different extent the illuminating optics beam path onto different entrance surfaces (7) of the prisms (4a, 4b, 5).

4. Illuminating device according to Claim 2, **characterized in that** the diaphragm (8) is constructed as an LCD which - being driven electronically - uncovers freely selectable fractions of the entrance surfaces (7) of the prisms (4a, 4b, 5).

5. Illuminating device according to one of the preceding claims, **characterized in that** at least one of the mirror surfaces (9) of at least one prism (4, 5) is designed as a totally reflecting surface, and **in that** at at least this mirror surface (9) at least one optically refracting part (10) is arranged such that it can, in a totally reflecting and refracting fashion, be displaced or pivoted or can be controlled electrooptically/electronically.

6. Illuminating device according to Claim 5, **characterized in that** the optically refracting part (10) is constructed as a transparent prism, as a glass plate or as an opaque component.

7. Illuminating device according to Claim 5 or 6, **characterized in that** the mirror surface (9) is arranged opposite a further mirror surface (12) which deflects beams emerging from the optically refractive part (10) at an angle to the main axis (6).

8. Illuminating device according to one of Claims 5 to 7, **characterized in that** the totally reflecting mirror surface (9) is formed at the second prism(s).

9. Illuminating device according to one of Claims 2-8, **characterized in that** in addition or alternatively a selective colour selection filter is provided in a fashion comparable to the diaphragm (8).

## Revendications

1. Dispositif d'éclairage pour un microscope chirurgical, avec un objectif principal (1) autour d'un axe principal (6), constitué d'une optique d'éclairage (2) dotée d'une source de lumière (3) et de deux éléments de renvoi disposés entre l'objectif principal (1) et l'optique d'éclairage (2) dont, en état de fonctionnement, le premier élément de renvoi a pour effet la déviation d'une partie du flux lumineux provenant de l'optique d'éclairage (2) sous un petit angle par rapport à l'axe principal (6) et le deuxième élément de renvoi a pour effet la déviation d'une autre partie du flux lumineux provenant de l'optique d'éclairage (2) sur l'axe principal (6), les éléments de renvoi étant prévus comme prismes (4, 5), le premier prisme (4a, 4b) du premier élément de renvoi étant réalisé en deux parties, trois prismes (4a, 4b, 5) étant ainsi prévus, les deux parties du premier prisme (4a, 4b) du premier élément de renvoi étant disposées symétriquement sur les côtés du deuxième prisme (5) du deuxième élément de renvoi pour réaliser l'éclairage symétrique de l'objet, les trois prismes (4a, 4b, 5) étant situés l'un à côté de l'autre dans un plan pour obtenir une hauteur finale réduite et les ouvertures d'entrée de lumière (7) dans les trois prismes (4a, 4b, 5) fermant ensemble la sortie de l'optique d'éclairage (2).

2. Dispositif d'éclairage selon la revendication 1, **caractérisé en ce qu'**au moins un diaphragme en iris (8) est disposé entre l'optique d'éclairage (2) et au moins une surface d'entrée (7) d'au moins l'un des prismes (4, 5).

3. Dispositif d'éclairage selon la revendication 2, **caractérisé en ce que** le diaphragme (8) est une plaque opaque dotée de fenêtres (11) qui peuvent être sélectionnées sélectivement et qui libèrent le parcours du rayon de l'optique d'éclairage sur différentes surfaces d'entrée (7) des prismes (4a, 4b, 5) dans une mesure qui varie en fonction des besoins.

4. Dispositif d'éclairage selon la revendication 2, **caractérisé en ce que** le diaphragme (8) est un dispositif à cristaux liquides LCD qui libère sous une commande électronique des parties librement sélectionnables des surfaces d'entrée (7) des prismes (4a, 4b, 5).

5. Dispositif d'éclairage selon l'une des revendications précédentes, **caractérisé en ce qu'**au moins l'une des surfaces réfléchissantes (9) d'au moins un prisme (4, 5) est configurée comme surface à réflexion totale et **en ce qu'**au moins une partie (10) de réfraction de lumière est disposée au moins sur cette surface réfléchissante (9) de manière à fournir une réflexion totale par déplacement, pivotement ou commande électro-optique ou électronique.

6. Dispositif d'éclairage selon la revendication 5, **caractérisé en ce que** la partie (10) de réfraction de la lumière est un prisme transparent, une plaque de verre ou un composant opaque.

7. Dispositif d'éclairage selon la revendication 5 ou 6, **caractérisé en ce que** la surface réfléchissante (9) est disposée face à une autre surface réfléchissante (12) qui dévie les rayons sortants de la partie (10) de réfraction de la lumière obliquement par rapport à l'axe principal (6).

8. Dispositif d'éclairage selon l'une des revendications 5 à 7, **caractérisé en ce que** la surface réfléchissante (9) à réflexion totale est réalisée sur le deuxième prisme (5).

9. Dispositif d'éclairage selon l'une des revendications 2 à 8, **caractérisé en ce qu'**en supplément ou en variante, un filtre de sélection de couleur sélectif est prévu de manière similaire au diaphragme (8).
